# EUROPEAN PATENT SPECIFICATION

(11) **EP 1 490 121 B1**
(45) Date of publication and mention of the grant of the patent: **04.11.2009**
(21) Application number: 03745663.9
(22) Date of filing: 28.03.2003
(51) Int. Cl.: A61L 24/04, A61L 24/00, A61L 27/50, A61L 27/16, A61L 31/14, A61L 31/04, A61K 9/16

(54) **DRUG DELIVERY PARTICLE**
PARTIKEL FÜR ARZNEISTOFFABGABE
PARTICULE D'ADMINISTRATION DE MEDICAMENTS

(30) Priority: 29.03.2002 US 109966; 04.04.2002 US 116330; 30.08.2002 US 232265
(43) Date of publication of application: 29.12.2004
(73) Proprietor: Boston Scientific Limited, St. Michael (BB)
(72) Inventor: LANPHERE, Janel, Pawtucket, RI 02860 (US); MCKENNA, Erin, Boston, MA 02215 (US); NAIMARK, Wendy, Cambridge, MA 02138 (US); BUISER, Marcia, Watertown, MA 02472 (US); MANGIN, Stephan, Ashland, MA 01721 (US)
(74) Representative: Altenburg, Udo
(86) International application number: PCT/US2003/009691
(87) International publication number: WO 2003/082360

(56) References cited:
- EP-A- 0 402 031
- EP-A- 0 730 847
- WO-A-01/70291
- DE-A- 10 026 620
- US-A- 5 571 182
- US-A1- 2001 051 670
- DATABASE WPI Section Ch, Week 199808 Derwent Publications Ltd., London, GB; Class A14, AN 1998-082801 XP002250507 & JP 09 316271 A (KURARAY CO LTD), 9 December 1997 (1997-12-09)

## Description

### TECHNICAL FIELD

This invention relates to a drug delivery particle.

### BACKGROUND

Therapeutic agents can be delivered systemically, for example, by injection through the vascular system or oral ingestion, or they can be applied directly to a site where treatment is desired. It is also often desirable that the therapeutic agent be delivered at desired dosages for an extended period of time.

US 2003/0211073 discloses compositions comprising macromers having a backbone comprising units having a 1,2-diol and/or 1,3-diol structure for tissue bulking and coating. JP 09 316271 describes the use of substantially spherical porous polymer particles comprising polysaccharide, active agent and polyvinyl alcohol. DE 100 26 620 relates to porous particles and microspheres and their application in the technique and medicine. WO 01/70291 relates to elastic, hydrophilic and substantially spherical microspheres useful for dermal augmentation and tissue bulking.

### SUMMARY

In a first aspect, the invention features a drug delivery device comprising a spherical polymer particle having a sphericity of 90% or more, the particles having a center region from the center of the particle to a radius of r/3 including pores having a size of 20 to 35 micron and containing a therapeutic agent and a body region from r/3 to 2r/3 and a surface region from 2r/3 to r surrounding the center region, wherein the center region has a greater number of pores having a size of 20 microns to 35 microns than the body region and the surface region, and wherein the body region has a greater number of pores having a size of 5 microns to 18 microns than the surface region, and wherein the body region has a greater number of pores having a size of 5 microns to 18 microns than the surface region, wherein the particle is obtainable by a method comprising:
- generating drops comprising a base polymer and a gelling precursor;
- removing of the gelling precursor; and
- combining the particles with a therapeutic agent.

In another aspect, the invention features a method of manufacturing a drug delivery particle according to any of claims 1 to 13, having a sphericity of 90% or more and having a porosity gradient, the particles including a center region from the center of the particle to a radius of r/3 with pores having a size of 20 to 35 micron and containing a therapeutic agent and a body region from r/3 to 2r/3 and a surface region from 2r/3 to r surrounding the center region, wherein the center region has a greater number of pores having a size of 20 microns to 35 microns than the body region and the surface region, and wherein the body region has a greater number of pores having a size of 5 microns to 18 microns than the surface region, comprising:
- generating drops comprising a base polymer and a gelling precursor;
- removing of the gelling precursor; and
- combining the particles with a therapeutic agent

In another aspect, the invention features the use of a composition, wherein said composition comprising a substantially spherical polymer particle obtainable by the method of claim 14 and having a sphericity of 90% or more, the particle comprising polyvinyl alcohol, and including a center region from the center of the particle to a radius of r/3 including pores having a size of 20 micron to 35 micron, a body region from r/3 to 2r/3 and a surface region from 2r/3 to r surrounding the center region, wherein the center region has a greater number of pores having a size of 20 microns to 35 microns than the body region and the surface region and a therapeutic agent carried by the particle, and wherein the body region has a greater number of pores having a size of 5 microns to 18 microns than the surface region for the manufacture of a medicament for a drug delivery system.

Embodiments may include one or more of the following. The particle comprises PVA. The PVA is 1,3 diol acetalized. The polymer is modified by graft polymerization. The particle includes a polysaccharide. The polysaccharide is alginate. The particle has a coating of polymer. The coating is erodable. The coating covers a drug disposed on the surface of the particle. The therapeutic agent is effective for treatment of cancer. The particle has a sphericity of about 90% or more. The particle has a diameter of about 1 cm or less. The device is a collection of particles.

Embodiments may also include one or more of the following. The method includes reacting the base polymer and removing the gelling compound. The method includes drying the particle and exposing the dried particle to therapeutic agent. The method includes combining therapeutic agent prior to generating said drops. The gelling compound is a polysaccharide. The gelling compound is alginate. The method includes contacting the drops with a gelling agent. The method the gelling agent is a divalent agent. The base polymer is PVA. The method includes reacting the PVA by acetalization. The PVA has a molecular weight of about 75,000 g/mole or greater. The method includes modifying the viscosity of the base polymer and gelling compound prior to forming said drops. The method includes modifying the viscosity by heating. The method includes forming said drops by vibratory nebulization.

Embodiments may also include one or more of the following. The administration is by percutaneous injection. The administration is by a catheter. The therapeutic agent is effective treatment of uterine fibroids. Particles are delivered directly into a tissue mass. Particles are delivered through a body lumen, e.g., a vasicular lumen. The particles can be used in embolic applications.

Embodiment may include one or more of the following advantages. A sustained, controlled-dosage release of therapeutic agents can be effected by a substantially spherical agent-containing particle that includes a reservoir region in its interior and a metering region surrounding the reservoir region which controls the release of the agent from the particle.

Other features, objects and advantages follow. For example, features of the particles, including sizes, pore profiles, compressibility, spherosicity, and composition and the methods for making and administering, follow and can be found in USSN [01194/442001].

### DESCRIPTION OF DRAWINGS

FIG. 1 is a schematic illustrating administration of drug delivery particles;
FIG. 2 is a cross-sectional schematic illustrating release of drug from a particle;
FIG. 3A is a light micrograph of a collection of particles, while Fig. 3B is a scanning election microscope (SEM) photograph of a particle surface and FIGS. 3C — 3E are cross-sections of particles.
FIG. 4A is a schematic of the manufacture of a particle while Fig. 4B is an enlarged schematic of region A in FIG. 4A.
FIG. 5 is a photograph of gel-stabilized drops;

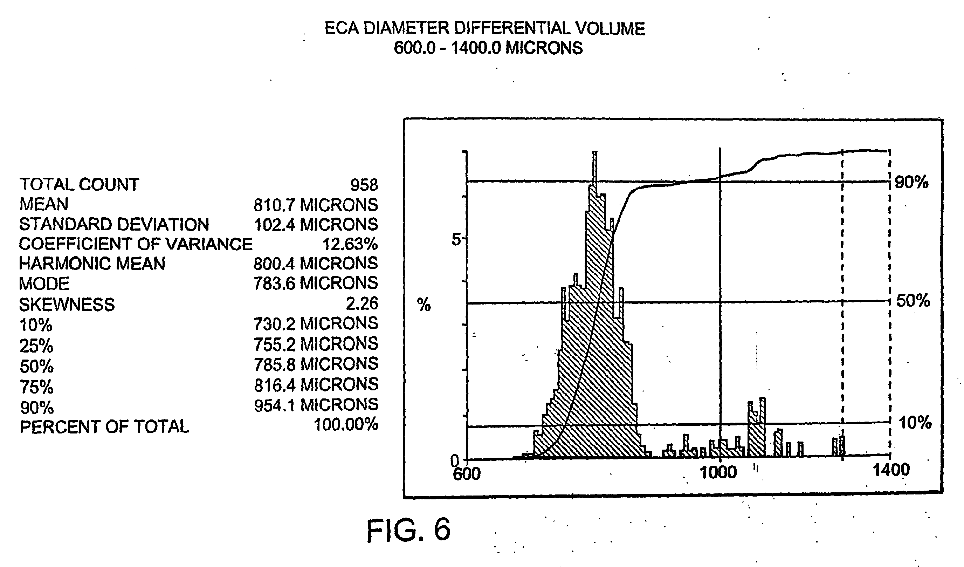

FIG. 6 is a graph of particles in uniformity.

### DETAILED DESCRIPTION

### Structure

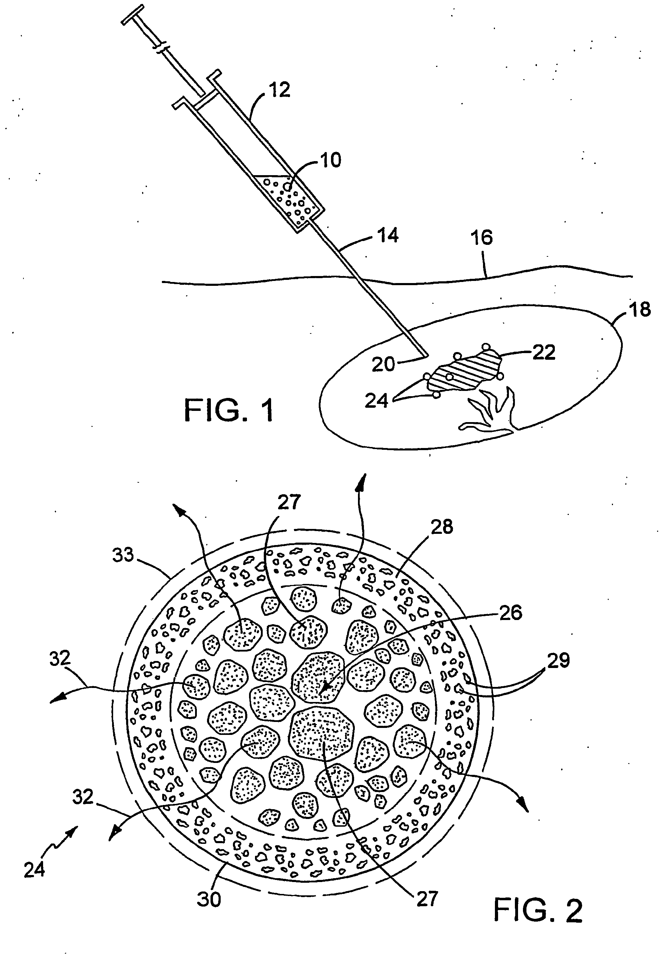

Referring to FIG. 1, a drug delivery composition 10 is injected using a syringe 12 with a needle 14 that is used to puncture the skin 16 and extend into the liver 18. The tip of the needle 20 is disposed within the tissue mass of the liver near and/or within a tumorous malignancy 22. The composition 10 includes a carrier fluid which carries drug delivery particles 24. The particles can be positioned about the lesion 22. In alternative embodiments, the particles can be delivered through the vasculature, e.g., by a catheter inserted into the hepatic artery. Another application includes treatment of uterine fibroids as described in USSN [01194/442001], supra.

Referring particularly to FIG. 2, the particles are substantially spherical and include an interior reservoir region 26 which is characterized by relatively large pores 27 and a metering region 28 which is characterized by relatively small pores 29. The large pores 27 in the reservoir region hold a supply of a therapeutic agent, such as a tumor-toxic agent, which diffuses through interpore passageways into the metering region and is released from the surface 30 of the particle (arrows 32) to expose adjacent tissue. The porous structure of a particle is believed to create a therapeutic agent concentration gradient from relatively high therapeutic concentration in the reservoir region to lower concentrations in the metering region. The relative size of the pores in the regions and the relative thickness of the metering region control the rate of elution of therapeutic agent from the particle. The substantially spherical shape of the particle contributes to symmetric elution in all directions. In addition, the relatively uniform thickness of the metering region surrounding the reservoir region enhances uniformity of elution dosage.

The particles are substantially formed of a highly water-insoluble, high molecular weight polymer. As will be discussed further below, a preferred polymer is high molecular weight polyvinyl alcohol (PVA) that has been acetalized. Preferably, the embolic particles are substantially pure intrachain 1,3 acetalized PVA and substantially free of animal derived residue such as collagen. In embodiments, the particles include a minor amount, e.g. less than about 0.2 weight %, of alginate or another polysaccharide or gelling material. The particle may also include an optional coating 33. The coating erodes in the body, e.g. on contact with body fluid, as will be discussed below.

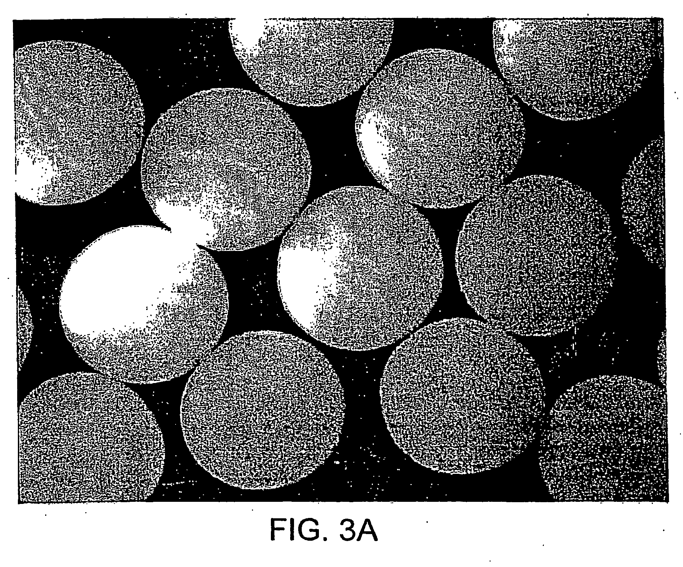

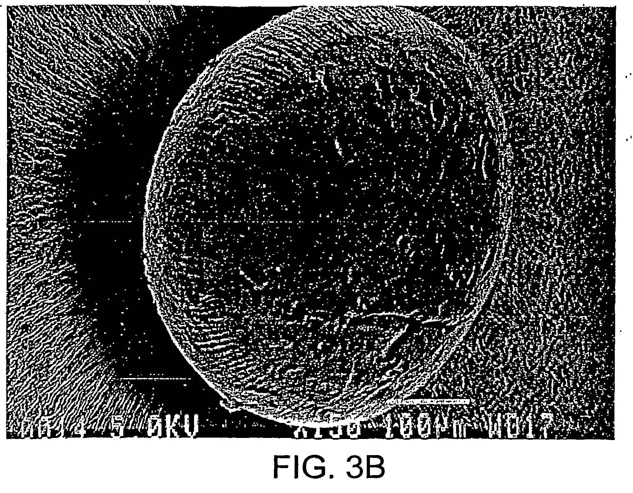

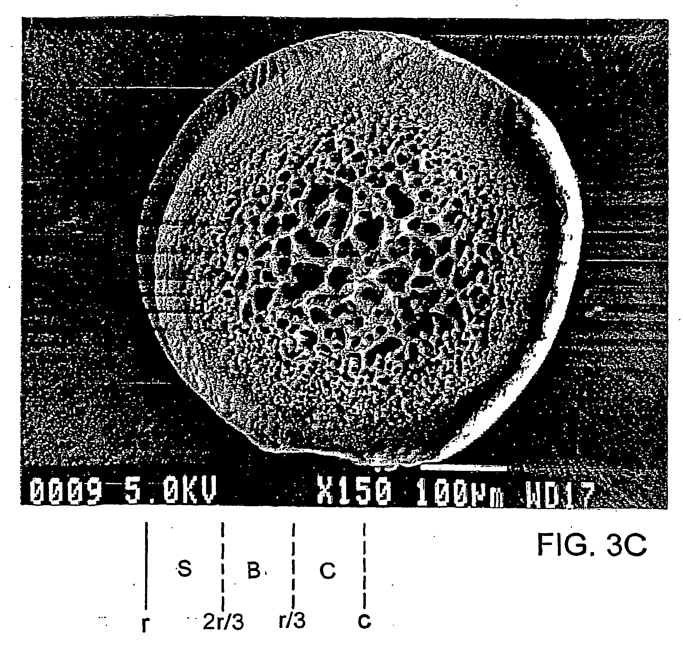

Referring to FIG. 3A, the particles have a substantially uniform shape and size. Referring to FIG. 3B, each particle has a well-defined outer spherical surface including relatively small, randomly located pores. Referring to FIGS. 3C - 3E, SEM images of cross-sections through the particle, the body defines pores which provide metering of therapeutic agent release, as well as compressibility and other properties.

In embodiments, the small pore region near the periphery of the embolic particle is relatively stiff and incompressible, which enhances resistance to shear forces and abrasion. In addition, the variable pore size profile produces a symmetric compressibility and, it is believed, a compressibility profile such that the particles are relatively easily compressed from a maximum, at rest diameter to a smaller, compressed first diameter but compression to even smaller diameter requires substantially greater force. A variable compressibility profile is believed to be due to the presence of a relative weak, collapsible inter-pore wall structure in the center region where the pores are large, and a stiffer inter-pore wall structure near the surface of the particle, where the pores are more numerous and relatively small. The variable pore size profile also is believed to enhance elastic recovery after compression. The pore structure also influences the density of the embolic particles and the rate of therapeutic agent and body fluid uptake.

The particles can be delivered through a syringe or a catheter. The size of the lumen of the syringe or the catheter can be larger than the particle diameter to reduce compression of the particles during delivery, which can eject therapeutic agent from the particle prematurely. While compression can result in release of therapeutic agent, the metering region can retard substantial release under low compression force. In embodiments, the particles are compressed during delivery in order to use a delivery device that has a small diameter to reduce patient trauma or more accurately position the particles about a lesion. The carrier fluid in which the particles are suspended can include therapeutic agent so that upon recovery to normal diameter, the agent is drawn into the pores of the particle. For example, the particles can be delivered through a catheter having a lumen area that is smaller, e.g. 50% smaller or less, than the uncompressed cross-sectional area of the particles. The compression force is provided indirectly by increasing the pressure applied to the carrier fluid by pressing the syringe plunger. The particles are relatively easily compressed to diameters sufficient for delivery into the body. The robust, rigid surface region resists abrasion when the embolic particles contact hard surfaces such as syringe surfaces, hard plastic or metal stopcock surfaces, and the catheter lumen wall (e.g. Teflon) during delivery. Once in the body, the particles recover to original diameter for efficient transport in the carrier and body fluid stream. At the point of occlusion, the particles can again compress as they aggregate in an occlusion region. The particles form a dense occluding mass. The compression in the body is limited and the number of embolic particles needed to occlude a given diameter may be reduced. The particles can also be delivered directly into a tissue mass where reexpansion to a larger diameter firmy lodges the particle into the tissue.

In embodiments, the particles have a diameter in the range of 1 cm or less, e.g., 5 mm to 1 mm or less, e.g., about 1200 microns or less, and about 10 microns or more, e.g. about 400 microns or more and the pores are about 50 or 35 to 0.01 micron. Preferably, the particles are classified in size ranges of about 500-700 microns, about 700-900 microns, or about 900-1200 microns. The particles have a mean diameter in approximately the middle of the range and variance of about 20% or less, e.g. 15% or 10% or less.

Referring specifically to FIG. 3C, the particles can be considered to include a center region, C, from the center of the particle to a radius of about r/3, a body region, B, from about r/3 to about 2 r/3 and a surface region, S, from 2r/3 to r. The regions can be characterized by the relative size of the pores and the number of pores of given sizes. In embodiments, the center region has a greater number of relatively large pores than the body region and the surface region. The large pores are in the range of about 20 micron or more, e.g. 30 micron or more, or in the range of about 20 to 35 micron. The body region has a greater number of intermediate size pores than the surface region. The intermediate size pores are in the range of about 5 to 18 micron. In embodiments, the regions may also have different densities, with the density of the surface region being greater than the density of the body region, and the density of the body region being greater than the density of the center region.

The size of the pores in each of the regions can also be characterized by a distribution. In embodiments, the predominant pore size(s) in the center region being greater than the predominant pore size(s) in the body region and the predominant pore size(s) in the body region is greater than the predominant pore size(s) in the surface region. In embodiments, in the predominant pore size in the center region is 20 micron or more, e.g. 30 microns or more, or in the range of 0 to 35 microns. The predominant pore size in the body region is about 18 micron or less, e.g. about 15 micron or less, or in the range of 18 to 2 micron. The pores in the surface region are preferably predominantly less than 1 micron, e.g. 0.1 to 0.01 micron.

In embodiments, the predominant pore size in the body region is 50 to 70% of the pore size in the center region and the pore size in the surface region is 10% or less, e.g. 2% of the pore size in the body region. The size of the pores on the outer surface of the particle is predominantly in the range of 1 micron or less, e.g. 0.1 or 0.01 micron. In embodiments, the surface and/or surface region is substantially free of pores having a diameter larger than 10 micron or larger than 1 micron. In embodiments, the predominant pore size is in the region 0.8 or 0.9r to r is 1 micron or less, e.g. 0.5 to 0.1 micron or less. The region from the center of the particle to 0.8 or 0.9r has pores of 10 micron or greater and/or has a predominant pore size of 2 to 35 micron. In embodiments, the predominant pore size in the region 0.8 or 0.9r to r is 5% or less, e.g. 1% or 0.3% or less than the predominant pore size in the region from the center to 0.9r. the largest pores in the particles can have a size in the range of 1% or 5% or 10% or more of the particle diameter.

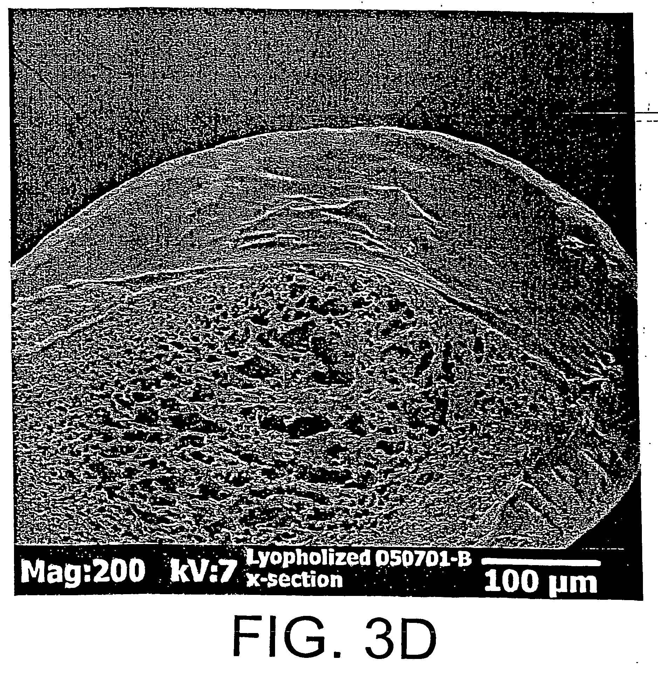

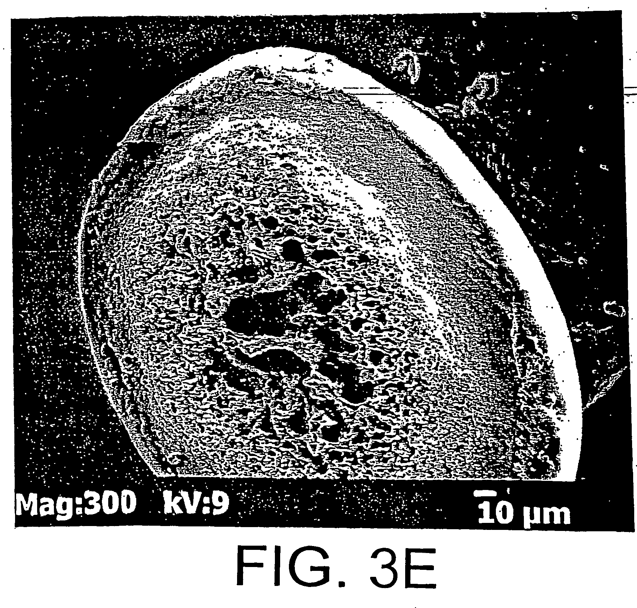

The size of the pores can be measured by viewing a cross-section as in Fig. 3C. For irregularly shaped pores, the maximum visible cross-section is used. The predominant pore size(s) can be found by measuring the size of the visible pores and plotting the number of pores as a function of size. The predominant pore size(s) are the sizes that are about the maximum in the distribution. In Fig. 3C, the SEM was taken on wet particles including absorbed saline, which were frozen in liquid nitrogen and sectioned. (Fig. 3B was taken prior to sectioning.) In Figs. 3D and 3E, the particle was freeze-dried prior to sectioning and SEM analysis.

The density of the particles is such that they are readily suspended in the carrier fluid such as a mixture of saline and contrast solution and remain suspended during delivery. In embodiments, the density is in 1.1 - 1.4g/cm3. For suspension in a saline-contrast solution, the density is 1.2 - 1.3g/cm³. The sphericity after compression in a catheter to 50% or more of their cross-sectional area is 0.90 or 0.95 or greater. In embodiments, the particles can be manually compressed, essentially flattened, while wet to less than 50% of original diameter and then, upon exposure to fluid, regain a sphericity of about 0.9 or more. The carrier fluid can be a pharmaceutically acceptable carrier such as saline or contrast agent or therapeutic agent or a combination of these carriers. The particles or composition can be sterilized.

### Manufacture

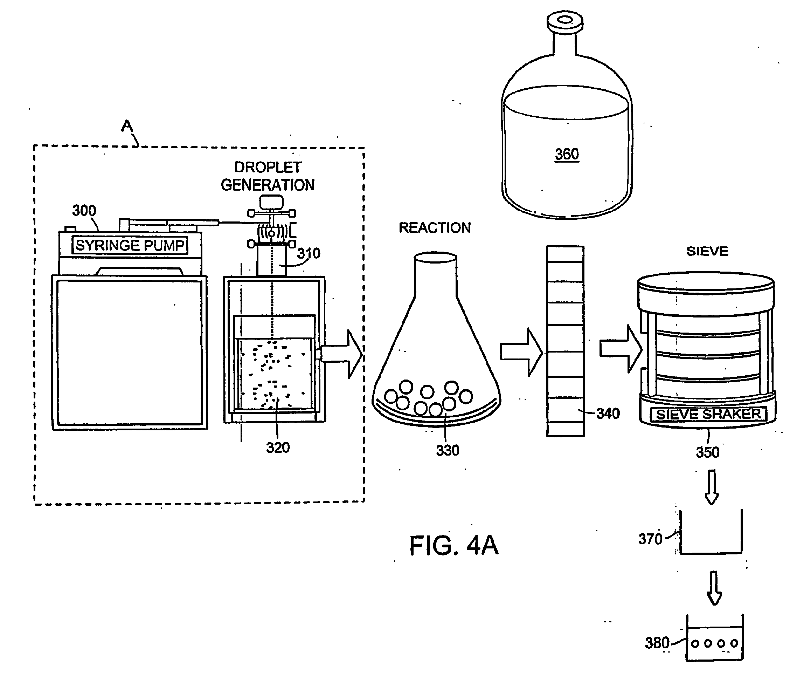

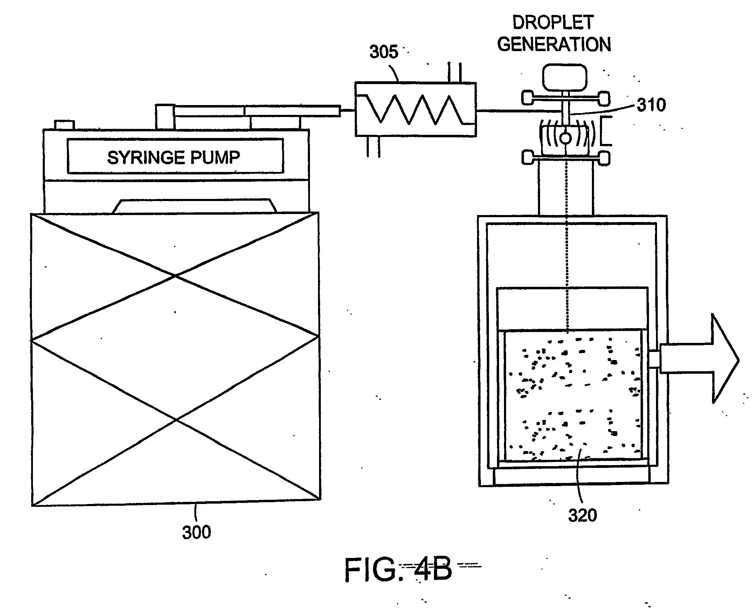

Referring to FIG. 4, a system for producing particles includes a flow controller 300, a drop generator 310, a gelling vessel 320, a reactor vessel 330, a gel dissolution chamber 340, a filter 350, a supply of therapeutic agent 360, a particle drying chamber 370, and a particle rehydrator vessel 380. The flow controller 300 delivers polymer solutions to a viscosity controller 305, which heats the solution to reduce viscosity prior to delivery to the drop generator 310. The drop generator 310 forms and directs drops into a gelling vessel 320, where drops are stabilized by gel formation. The gel-stabilized drops are transferred from the gelling vessel 320 to reactor vessel 330 where the polymer in the gel-stabilized drops are reacted forming precursor particles. The precursor particles are transferred to a gel dissolution chamber 340, where the gel is dissolved. The particles are then filtered in a filter 350 to remove debris, sterilized, and packaged as a composition including the particles. As will be discussed below, the therapeutic agent can be incorporated into the particles at various stages. In the embodiment illustrated, after filtering, the particles can be dried in a chamber 370, e.g. under vacuum (e.g., by lyophilization) with or without heat application or air dried with or without heat, e.g., at room temperature. The dried particles are then rehydrated in a vessel 380 which includes therapeutic agent. In the rehydration process, the therapeutic agent is drawn into the particles through the pore structure. The particles can then be packed in a solution of therapeutic agent. The particles can be mixed with saline or contrast agent at the time of administration.

A base polymer and a gelling precursor are dissolved in water and mixed. The mixture is introduced to a high pressure pumping apparatus, such as a syringe pump (e.g., model PHD4400, Harvard Apparatus, Holliston, MA). Examples of base polymers include polyvinyl alcohol, polyacrylic acid, polymethacrylic acid, poly vinyl sulfonate, carboxymethyl cellulose, hydroxyethyl cellulose, substituted cellulose, polyacrylamide, polyethylene glycol, polyamides, polyureas, polyurethanes, polyester, polyethers, polystyrene, polysaccharide, polylactic acid, polyethylene, polymethylmethacrylate and copolymers or mixtures thereof. A preferred polymer is polyvinyl alcohol. The polyvinyl alcohol, in particular, is hydrolyzed in the range of 80 to 99%. The weight average molecular weight of the base polymer can be in the range of 9000 to 186,000, 85,000 to 146,000 or 89,000 to 98,000. Gelling precursors include, for example, alginates, alginate salts, xanthan gums, natural gum, agar, agarose, chitosan, carrageenan, fucoidan, furcellaran, laminaran, hypnea, eucheuma, gum arabic, gum ghatti, gum karaya, gum tragacanth, hyalauronic acid, locust beam gum, arabinogalactan, pectin, amylopectin, other water soluble polysaccharides and other ionically crosslinkable polymers. A particular gelling precursor is sodium alginate. A preferred sodium alginate is high guluronic acid, stem-derived alginate (e.g. 50 or 60% or more guluronic acid with a low viscosity e.g. 20 to 80 cps at 20°C) which produces a high tensile, robust gel. High molecular weight PVA is dissolved in water by heating, typically above about 70°C, while alginates can be dissolved at room temperature. The PVA can be dissolved by mixing PVA and alginate together in a vessel which is heated to autoclave temperature (about 121°C). Alternatively, the PVA can be disposed in water and heated and the alginate subsequently added at room temperature to avoid exposing the alginate to high temperature. Heat can also be applied by microwave application. For PVA/alginate, the mixture is typically 7.5 to 8.5%, e.g. 8% by weight PVA and about 1.5 to 2.5%, e.g. about 2%, by weight alginate.

Referring to FIG. 4B, the viscosity controller 305 is a heat exchanger circulating water at a predetermined temperature about the flow tubing between the pump and drop generator. The mixture of base polymer and gelling precursor flows into the viscosity controller 305, where the mixture is heated so that its viscosity is lowered to a level for efficient formation of very small drops. For a high molecular weight PVA/alginate solution, the temperature of the circulating water is less than about 75°C and more than about 60°C, for example, 65°C which maintains the mixture at a viscosity of 90-200 mPa.s. For spherical particles, the viscosity of the drops is maintained so they are captured in the gelling vessel without splintering or cojoining which can create irregular, fiberous particles. In other embodiments, the flow controller and/or the drop generator can be placed in a temperature-controlled chamber, e.g. an oven, or a heat tape wrap, to maintain a desired viscosity.

The drop generator 310 generates substantially spherical drops of predetermined diameter by forcing a stream of the mixture of base polymer and gelling precursor through a nozzle which is subject to a periodic disturbance to break up the jet stream into drops. The jet stream can be broken into drops by vibratory action generated for example, by an electrostatic or piezoelectric element. The drop size is controlled by controlling the flow rate, viscosity, amplitude, and frequency at which the element is driven. Lower flow rates and higher frequencies produce smaller drops. A suitable electrostatic drop generator 310 is available from NISCO Engineering, model NISCO Encapsulation unit VAR D, Zurich, Switzerland. In embodiments, the frequency is in the range of 0.1 to 0.8 kHz. The flow rate through the droplet generator is in the range of 1 to 12 mL per minute. The drop generator can include charging the drops after formation such that mutual repulsion between drops prevents drop aggregation as they travel from the generator to the gelling vessels. Charging may be achieved by, e.g. an electrostatic charging device such as a charged ring positioned downstream of the nozzle.

Drops of the base polymer and gelling precursor mixture are captured in the gelling vessel 320. The gelling vessel 320 contains a gelling agent which interacts with the gelling precursor to stabilize drops by forming a stable gel. Suitable gelling agents include, for example, a divalent cation such as alkali metal salt, alkaline earth metal salt or a transition metal salt that can ionically crosslink with the gelling agent. An inorganic salt, for example, a calcium, barium, zinc or magnesium salt can be used as a gelling agent. In embodiments, particularly those using an alginate gelling precursor, a suitable gelling agent is calcium chloride. The calcium cations have an affinity for carboxylic groups in the gelling precursor. The cations complex with carboxylic groups in the gelling precursor resulting in encapsulation of the base polymer in a matrix of gelling precursor.

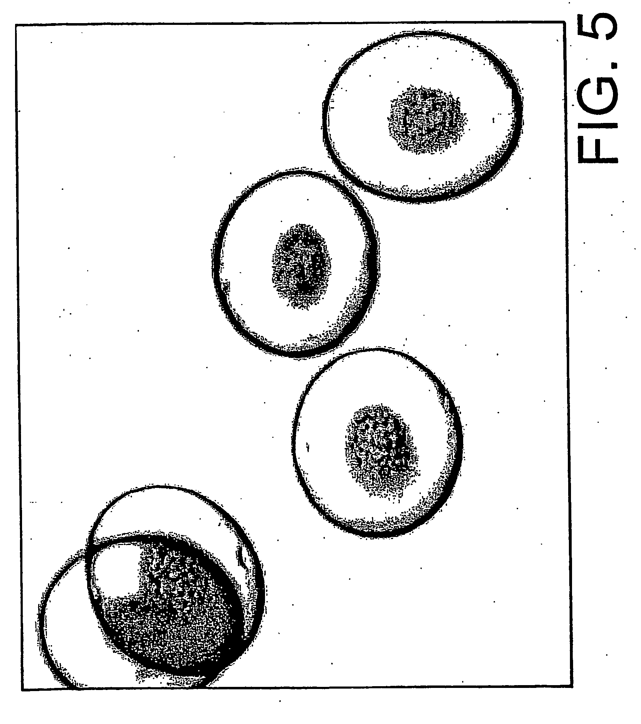

Referring to FIG. 5, a photo-image of the gelled particles, the gelling agent is in an amount selected in accordance with the desired properties of the particles. A pore structure in the center of the particle forms in the gelling stage. The concentration of the gelling agent can control void formation in the embolic particle, thereby controlling the porosity gradient in the embolic particle. Adding non-gelling ions, for example, sodium ions, to the gelling solution can limit the porosity gradient, resulting in a more uniform intermediate porosity throughout the particle. In this manner the thickness and pore profile of the metering region can be controlled. In embodiments, the gelling agent is, for example, 0.01-10 weight percent, 1-5 weight percent or 2 weight percent in deionized water.

Following drop stabilization, the gelling solution is decanted from the solid drops and the stabilized drops are transferred to the reactor vessel 330. In the reactor vessel 330, the stabilized drops are reacted to produce precursor particles. The reactor vessel includes an agent that chemically reacts with the base polymer, e.g. to cause crosslinking between polymer chains and/or within a polymer chain. The agent diffuses into the stabilized drops from the surface of the particle in a gradient which, it is believed, provides more crosslinking near the surface of the stabilized drop compared to the body and center of the drop. Reaction is greatest at the surface of the drop, providing a stiff, abrasion resistant exterior. For polyvinyl alcohol, for example, the vessel 330 includes aldehydes, such as formaldehyde, glyoxal, benzaldehyde, aterephthalaldehyde, succinaldehyde and glutaraldehyde for the acetalization of polyvinyl alcohol. The vessel 330 also includes an acid, for example, strong acids such as sulfuric acid, hydrochloric acid, nitric acid and weak acids such as acetic acid, formic acid and phosphoric acid. In embodiments, the reaction is primarily a 1,3 acetalization:

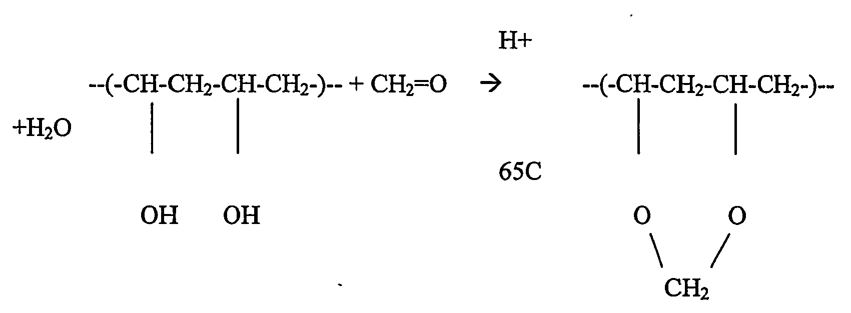

This intra-chain acetalization reaction can be carried out with relatively low probability of inter-chain crosslinking as described in John G. Pritchard "Poly(Vinyl Alcohol) Basic Properties And Uses (Polymer Monograph, vol. 4) (see p. 93-97), Gordon and Breach, Science Publishers LTD., London, 1970, the entire contents of which is hereby incorporated by reference. Some OH groups along a polymer chain may remain unconverted since the reaction proceeds in a random fashion and there will be left over OH groups that do not react with adjacent groups.

Adjusting the amount of aldehyde and acid used, reaction time and reaction temperature can control the degree of acetalization. In embodiments, the reaction time is e.g., 5 minutes to 1 hour, 10 to 40 minutes or 20 minutes. The reaction temperature can be 25°C to 150°C or 75°C to 130°C or 65°C. The reactor vessel is placed in a water bath fitted with a orbital motion mixer. The crosslinked precursor particles are washed several times with deionized water to neutralize the particles and remove any residual acidic solution.

The precursor particles are transferred to the dissolution chamber 340 to remove the gelling precursor, e.g. by an ion exchange reaction. In embodiments, sodium alginate is removed by ion exchange with a solution of sodium hexa-metaphosphate (EM Science). The solution can include, for example, ethylenediaminetetracetic acid (EDTA), citric acid, other acids and phosphates. The concentration of the sodium hexa-metaphosphate can be, for example, 1-20 weight %, 1-10 weight % or 5 weight % in deionized water. Residual gelling precursor, for example, sodium alginate, can be determined by an assay for detection of uronic acids in, for example, alginates containing mannuronic and guluronic acid residues. Residual alginate, for example, may be present in the range of 20-35% by weight prior to rinsing and in the range of 0.01-0.5% or 0.1-0.3% or 0.18% in the particles after rinsing for 30 minutes in water at about 23°C.

The particles are filtered through filter 350 to remove residual debris. Particles of 500 to 700 microns are filtered through a sieve of 710 microns and then a sieve of 300 microns. Particles of 700 to 900 microns are filtered through a sieve of 1000 microns and then a sieve of 500 microns. Particles of 900 to 1200 microns are filtered through a sieve of 1180 microns and then a sieve of 710 microns.

The filtered particles are sterilized by a low temperature technique such as e-beam irradiation, and packaged, typically 1 to 5 ml of particles in 5 to 10 ml saline. In embodiments, electron beam irradiation can be used to pharmaceutically sterilize the particles to reduce bioburden. In e-beam sterilization, an electron beam is accelerated using magnetic and electric fields, and focused into a beam of energy. This resultant beam can be scanned by means of an electromagnet to produce a "curtain" of accelerated electrons. The accelerated electron beam penetrates the collection of embolic particles to confer upon them electrons which destroy bacteria and mold to sterilize and reduce the bioburden in the embolic particles. Electron beam sterilization can be carried out by sterilization vendors such as Titan Scan, Lima, Ohio.

The therapeutic agent can be incorporated in the particle at various stages. As discussed above, the agent may be added to the particle after particle formation. For example, the particle can be dried and rehydrated with the therapeutic agent or a solution including the therapeutic agent. Alternatively, the therapeutic agent can be added during particle formation. For example, the agent can be mixed with PVA and alginate upstream of droplet formation or after droplet formation in the gelling vessel, reaction vessel, or dissolution chamber or in a separate step after any of these stages. The particles may also be used to deliver therapeutic agent at the stabilized drop stage without cross-linking the base polymer or at the precursor particle stage with crosslinked base polymer with or without removing the gelling precursor or gelling agent. Alternatively, the therapeutic agent can be provided only to the surface and/or metering region, e.g., by coating particle, without including substantial amounts of agent in the interior portions of the particle, e.g., the reservoir region.

The particles can be coated to include high concentration of therapeutic agent on their surface. The agent on the surface can release an initial dosage of agent while agent in the body of the particle provides a prolonged dosage over the extended period of time. The agent on the surface can be the same or different from the agent in the body of the particle. The agent on the surface can be applied by exposing the particle to a high concentration solution of the agent. The agent coated particle can include another coating over the surface the therapeutic agent, e.g., a degradable polymer which erodes when the particle is administered or meters drug out flow from the surface, e.g., by providing a porous membrane. The coating can delay an initial burst of drug release. The coating can be applied by dipping or spraying the particle. The erodable polymer could be a polysaccharide, such as an alginate. Suitable material for alginate coatings are described in Edwards-Levy Biomaterials 1999, Nov 20 (21) 2069-84; J. Microencapsol. 1999 May-June 16(3); 291-301; and Takka et al. J. Microencapsol. 1999 May-June 16(3), 275-90. Other erodable coatings include water soluable polymers such as polyvinyl alcohol, e.g., that has not been cross-linked. Other coatings include biodegradeable poly DL-lactide-poly ethylene glycol (PELA) discussed in Zhou et al. J. Control Release 2001 Jul. 10; 75;(1-2):27-36 or gelatin as discussed in Huang et al. Int. J. Pharm 1995 May 10 182(1):93-100. Other coatings include hydrogels such as polyacrylic acid, haluronic acid, gelatin, or carboxymethyl cellulose. Other coatings include polyethylene glycols (PEG), chitosan, polyesters such as polycaproloctones, and poly(de-lactic coglycolic acid (PLGA). Suitable coatings of these types are discussed in J. Control Release, vol. 78, 1-3, 17 Jan. 2002, pp. 15-24. The coatings can include therapeutic agent or be substantially free of therapeutic agent. The therapeutic agent in the coating can be the same or different as an agent on a surface layer of the particle and/or within the particle. A polymer coating, e.g. an erodable coating, can be applied to the particle surface in cases where a high concentration of drug has not been applied to the particle surface. The fluoroscopic visibility of the particle can be enhanced by incorporating a highly radiopaque material such as a metal, e.g. tantalum or platinum into the polymer matrix of the particle or the coating

The particles can be modified by chemical or physical modifications that affect attachment and/or release of the therapeutic agent, the visibility of the particles, or their shape. For example, the polymer of the particle can be modified by graft polymerization to, for example, provide a reactive side chain. A therapeutic agent is attached covelantly or ionically to the reactive moiety of the graft polymer. A polymer that is grafted to the particle can be further polymerized to influence polymer chain length to create a molecular level morphology or vary hydrophobicity. Suitable graft polymers include polymers with carboxylic acid, anhydride, or aceto-acetyl groups which can be grafted to, e.g. PVA side groups modified to provide acrylic acids. Graft polymerization is discussed in Biomaterials, 2002 Feb. 23 (3) 863-71 and "Polyvinyl Alcohol Developments," Ed. C.A. Finch, John Whiley, 1992 (see especially sections 6.2.3 and 7.3.1). Suitable graft polymers also include peptides that include cell binding domains. Examples are discussed in Hubbell, Biomacromolecules, 2002, vol. 3, 710-23. Species capable of cell membrane penetrations e.g. polyluecine oligomer can be attached to the particle to enhance cell attachment. Targeting ligonds such as galactos can be introduced onto the surface of a particle. Galactos attachment onto polymers is discussed in Biotechnology Bioengineering, 2002 April 5 (78) 1-10. The grafted segment can be provided with reactive moieties such as amines, carboxylic acids or thiols to which therapeutic agent can be attached. The moieties can be used to modify the hydrophobic/hydrophilic and catonic/anionic nature of the particle surface. An example of a polymer that can be grafted is poly(vinyl alcohol)-graft-poly(lactic-coglycolic acid) to produce brush-like branched polyesters for enhancing protein release, as discussed in Frauke-Pistel et al. J. Control Release 2001 May 18; 73(1):7-20. Particle charged and hydrophobicity can be modified by grafting. For example, a negatively charged hydrophilic backbone poly (2-sulfobutyl vinyl alcohol)-g-poly(lactide-co-glycolide) is described in Jung et al. J. Control Release 2000 Jul 3; 67(2-3):157-69.

The polymer of the particle can also be modified by, e.g. block copolymerization to provide reactive moieties for graft polymerization and/or for direct therapeutic agent attachment. The polymer can also be modified to provide reactive groups at specific sites. For example, hydroxyl groups of PVA can be modified to provide more reactive sites, such as e.g. amines, carboxylic acids, or thiols.

Release kinetics can also be modified by controlling crosslinking. Techniques for crosslinking PVA and controlling release kinetics are discussed in Kim et al. Pharmaceutical Research, vol. 9, No. 1 (1992); Cosmetic and Pharm. App. For Polymers, Aug. 1990 p.709-14; and Polymer Mater. Sci. Eng. (1990) vol. 63, p. 64-7. Crosslinking is also described in A.R. Bachtsi and C. Kiparissides Journal of Microencapsulation, 1995, vol. 12 part 1, p. 23-35; Tobata et al. J. Control Release vol. 50, part 1-3, p. 123-133; and Orenti et al,. Arch. Pharm (Weinheim) 2000 Dec: 333 (12), 421-4 and Sappimath et al., J.Biomat. Sci. Polym. Ed. 2000j 11(i); 27-43.

The shape of the particles can be modified by physical deformation followed by crosslinking as described in USSN 10/116,330 filed April 14, 2002.

The particles can be coated on or incorporated into other medical devices, such as implantable devices including stents, embolization coils, arterial filters, artificial heart valves, catheters, and balloons such as angioplasty balloons. Other medical delivery includes wound dressings.

### Therapeutic Agents and Use

Therapeutic agents include materials that are biologically active to treat physiological condition. The agent can be active in release from the particle to tissue or active as it resides in the particle and is exposed to tissue or body fluid in communication with the particle.

The term "therapeutic agent" includes one or more "therapeutic agents" or "drugs". The terms "therapeutic agents" and "drugs" are used interchangeably and include pharmaceutically active compounds, nucleic acids with and without carrier vectors such as lipids, compacting agents (such as histones), virus (such as adenovirus, adeno-associated virus, retrovirus, lentivirus and a-virus), polymers, hyaluronic acid, gene therapies, proteins, cells, stem cells and the like, or combinations thereof, with or without targeting sequences.

Specific examples of therapeutic agents include, for example, pharmaceutically active compounds, proteins, cells, stem cells, oligonucleotides, ribozymes, antisense oligonucleotides, DNA compacting agents, gene/vector systems (i.e., any vehicle that allows for the uptake and expression of nucleic acids), nucleic acids (including, for example, recombinant nucleic acids; naked DNA, cDNA, RNA; genomic DNA, cDNA or RNA in a noninfectious vector or in a viral vector and which further may have attached peptide targeting sequences; antisense nucleic acid (RNA or DNA); and DNA chimeras which include gene sequences and encoding for ferry proteins such as membrane translocating sequences ("MTS") and herpes simplex virus-1 ("VP22")), and viral, liposomes and cationic and anionic polymers and neutral polymers that are selected from a number of types depending on the desired application. Non-limiting examples of virus vectors or vectors derived from viral sources include adenoviral vectors, herpes simplex vectors, papilloma vectors, adeno-associated vectors, retroviral vectors, and the like. Non-limiting examples of biologically active solutes include anti-thrombogenic agents such as heparin, heparin derivatives, urokinase, and PPACK (dextrophenylalanine proline arginine chloromethylketone); antioxidants such as probucol and retinoic acid; angiogenic and anti-angiogenic agents and factors; agents blocking smooth muscle cell proliferation such as rapamycin, angiopeptin, and monoclonal antibodies capable of blocking smooth muscle cell proliferation; anti-inflammatory agents such as dexamethasone, prednisolone, corticosterone, budesonide, estrogen, sulfasalazine, acetyl salicylic acid, and mesalamine; calcium entry blockers such as verapamil, diltiazem and nifedipine; antineoplastic/antiproliferative/anti-mitotic agents such as paclitaxel, 5-fluorouracil, methotrexate, doxorubicin, daunorubicin, cyclosporine, cisplatin, vinblastine, vincristine, epothilones, endostatin, angiostatin and thymidine kinase inhibitors; antimicrobials such as triclosan, dephalosporins, aminoglycosides, and nitorfurantoin; anesthetic agents such as lidocaine, buplvacaine, and ropivacaine; nitrix oxide (NO) donors such as lisidomine, molsidomine, L-argine, NO-protein adducts, NO-carbohydrate adducts, polymeric or oligomeric NO adducts; anti-coagulants such as D-Phe-Pro-Arg chloromethyl ketone, an RGD peptide-containing compound, heparine, antithrombin compounds, platelet receptor antagonists, anti-thrombin antibodies, anti-platelet receptor antibodies, enoxaparin, hirudin, Warafin sodium, Dicumarol, aspirin, prostaglandin inhibitors, platelet inhibitors and tick antiplatelet factors; vascular cell growth promoters such as growth factors, growth factor receptor antagonists, transcriptional activators, and translational promoters; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; agents which interfere with endogenous vascoactive mechanisms; survival genes which protect against cell death, such as anti-apoptotic Bcl-2 family factors and Akt kinase; and combinations thereof. Cells can be of human origin (autologous or allogenic) or from an animal source (xenogeneic), genetically engineered if desired to deliver proteins of interest at the injection site. The delivery mediated is formulated as needed to maintain cell function and viability. Any modifications are routinely made by one skilled in the art.

Useful polynucleotide sequences include DNA or RNA sequences having a therapeutic effect after being taken up by a cell. Examples of therapeutic polynucleotides include anti-sense DNA and RNA; DNA coding for an anti-sense RNA; or DNA coding for tRNA or rRNA to replace defective or deficient endogenous molecules. The polynucleotides can also code for therapeutic proteins or polypeptides. A polypeptide is understood to be any translation product of a polynucleotide regardless of size, and whether glycosylated or not. Therapeutic proteins and polypeptides include as primary example, those proteins or polypeptides that can compensate for defective or deficient species in an animal, or those that act through toxic effects to limit or remove harmful cells from the body. In addition, the polypeptides or proteins that can be injected, or whose DNA can be incorporated, include without limitation, angiogenic factors and other molecules competent to induce angiogenesis, including acidic and basic fibroblast growth factors, vascular endothelial growth factor, hif-1, epidermal growth factor, transforming growth factor α and β, platelet-derived endothelial growth factor, platelet-derived growth factor, tumor necrosis factor α, hepatocyte growth factor and insulin like growth factor; growth factors; cell cycle inhibitors including CDK inhibitors, anti-restenosis agents, including p15, p16, p18, p19, p21, p27, p53, p57, Rb, nFkB and E2F decoys, thymidine kinase ("TK) and combinations thereof and other agents useful for interfering with cell proliferation, including agents for treating malignancies; and combinations thereof. Still other useful factors, which can be provided as polypeptides or as DNA encoding these polypeptides, include monocyte chemoattractant protein ("MCP-1"), and the family of bone morphogenic proteins ("BMP's"). The known proteins include BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Therapeutic agents include one or more of the following therapeutic agents: cells, stem cells, virus, protein, drug, enzymes, or combinations thereof.

Organs and tissues that may be treated include any mammalian tissue or organ, whether injected *in vivo* or *ex vivo.* Non-limiting examples include heart, lung, brain, liver, skeletal muscle, smooth muscle, kidney, bladder, intestines, stomach, pancreas, ovary, prostate, eye, tumors, cartilage and bone.

Other examples of therapeutic agents include the following.

Immunologic species such as antigens captured from specific cell lines (e.g. cancerous) can be absorbed/adsorbed or attached to surface of a particle, which can then be injected at the targeted cell mass, tissue or organ, e.g. a cancer site, to begin an immunologic reaction/cascade/response. Examples include HuRx, and DCVax from Northwest BioTherapeutics Inc., Bothell, Washington. An antigen or genetically engineered molecule can also be used. For example, anti-EGF receptor antibodies which help lengthen the time chemotherapy can be used as a treatment for colorectal cancer can be used. Examples include Cetuximab from ImClone Systems, New York, New York. Antibodies or receptors, genetically engineered or not, can also be used. Monoclonal antibodies to cells of blood vessels interact in the angiogenesis cascade, which is important for the growth of tumors can be used.

Radioactive molecules for radiopacity and/or treatment for cancer may be absorbed/adsorbed or attached to the surface of PVA particulates. Examples include radioactive materials such as iodine (131), gold or ytetrium.

Proteins required for signaling pathways may be absorbed/adsorbed or attached to the surface of the particulate including antibodies, antigens, monclonal antibodies, proteins found on cancer cells, proteins found on diseased cells in any system, proteins found on normal, nondiseased state cells in any system, or others. Signaling pathways of interest include pathways for cell regeneration, for cell death, for angiogenesis, for cell growth, for chronic heart failure, for cell differentiation or others. Suitable proteins include platelet derived growth factor BB as described in Bourke, 2002 Society for Biomaterials 28th Annual Meeting Transactions, page 144. Another particular therapeutic agent is vascular endothelial growth factor (VEGF) for enhancing endothelialization as described in J. Control Release, 2001, May 14, 14:72(1-3):101-13.

Complete whole cells, pieces of cells, genetically engineered cells or cells made of components of more than one organism may be attached to the surface of the particulate. Treatment includes diabetes or any disease caused by the cells of that organ lacking in producing a specific hormone/protein/organic molecule, cancer or Alzheimer's disease or diseases caused by the cells producing an incorrect product that is not in their function to create.

Antimicrobial coatings could coat the surface of the PVA particulate to aid in lessening infection/immunologic response to the presence of these products in the body. Coatings include the use of zinc, silver, iodine, triclosan and/or ciprofloxacin in a resin/polymer such as polyurethane.

Antigrowth drugs for cancer treatment may be absorbed/adsorbed or attached to the surface of the particle. Examples include Herceptin and Gleevec from Genetech and Novartis respectively. Small molecule chemotherapy drugs for targeted cancer treatment. Examples include, Ethiodol, Doxorubicin, Cisplatin and Mitomycin-C.

Particular therapeutic agents for treatment of liver tumors include agents used in chemoembolization, such as carboplatin, cisplatin, doxorobicin, mytomycinc and ethiodol, as discussed in Jean-Francois Geschwind, Dimitri Artemov et al., Journal of Vascular Interventional Radiology (2000) 11:1245-1255; Dheeraj Rajan, Michael Soulen et al, Journal of Vascular Interventional Radiology (2001) 12:187-193; and Leung, Goin and Sickies et al., Journal of Vascular Interventional Radiology (2001) 12:321-326. A particular tumor-toxic agent e.g. for liver treatment is paclitaxol, available from Bristol-Meyers Squib, New York, New York.

Particular therapeutic agents useful for treatment of uterine fibroid tumors include nonsteriodal anti-inflammatory medication, oral contraceptives, progestins, and gonadotrophin-releasing hormone agonists which may cause fibroid tumors to shrink as described in Levy et al., Journal of Women's' Imaging_2(4):168-175, 2000. Other therapeutic agents for uterine fibroid shrinkage include lupron, as discussed in Lipman, Appl. Radiol. 29(7):15-20, 2000.

Therapeutic agent may also include agents which bind to specific biological environments. The agents could, for example be placed on the exterior of the particle to make the particle targetable. The particles can be used for oral or topical administration as well as percutaneous administration. The particles can be used in chemoembolization in which drug is injected to a site and the particles are used to embolize the vasculature. The particles can include the same or a different agent or no agent. The particles can be used in combination with hydrogel based aneurysm embolization systems as described in Cruise et al., 2002, Society for Biomaterials 28th Annual Meeting-Transactions, page 203. Other applications include drug delivery for treatment of anyeurums, coronary artery disease, restenosis and benign prostatic hyperplasia, e.g. in combination with medical devices such as stents.

### Example

Particles are manufactured from an aqueous solution containing 8 weight % of polyvinyl alcohol, 99+% hydrolyzed, average M_{w} 89,000-120,000 (ALDRICH) and 2 weight% of gelling precursor, sodium alginate, PRONOVA UPLVG, (FMC BioPolymer, Princeton, NJ) in deionized water and the mixture is heated to about 121° C. The solution has a viscosity of about 310 centipoise at room temperature and a viscosity of about 160 cps at 65°C. Using a syringe pump (Harvard Apparatus), the mixture is fed to drop generator (Nisco Engineering). Drops are directed into a gelling vessel containing 2 weight % of calcium chloride in deionized water and stirred with a stirring bar. The calcium chloride solution is decanted within about three minutes to avoid substantial leaching of the polyvinyl alcohol from the drops into the solution. The drops are added to the reaction vessel containing a solution of 4% by weight of formaldehyde (37 wt% in methanol) and 20% by weight sulfuric acid (95-98% concentrated). The reaction solution is stirred at 65°C for 20 minutes. Precursor particles are rinsed with deionized water (3 X 300 mL) to remove residual acidic solution. The sodium alginate is substantially removed by soaking the precursor particles in a solution of 5 weight % of sodium hexa-methaphosphate in deionized water for 0.5 hour. The solution is rinsed in deionized water to remove residual phosphate and alginate. The particles are filtered by sieving, as discussed above, placed in saline (USP 0.9% NaCl) and followed by irradiation sterilization.

Particles were produced at the nozzle diameters, nozzle frequencies and flow rates (amplitude about 80% of maximum) described in Table I.

**TABLE 1**

| Bead Size (microns) | Nozzle Diameter (microns) | Frequency (kHz) | Flow Rate (mL/min) | Density (g/mL) | Sphericity | Suspendability (minutes) |
|---|---|---|---|---|---|---|
| 500-700 | 150 | 0.45 | 4 | - | 0.92 | 3 |
| 700-900 | 200 | 0.21 | 5 | 1.265 | 0.94 | 5 |
| 900-1200 | 300 | 0.22 | 10 | - | 0.95 | 6 |

Suspendability is measured at room temperature by mixing a solution of 2 ml of particles in 5 ml of saline and 5 ml of contrast solution (Omnipaque 300, Nycomed, Buckinghamshire, UK) and observing the time for about 50% of the particles to enter suspension, i.e. not sink to the bottom or float to the top of a container (about 10 ml, 25 mm dia vial). Suspendability provides a practical measure of how long the particles will remain suspended in use. (Omnipaque is an aqueous solution of Iohexol, N.N.-Bis (2,3-dihydroxypropyl)-T-[N-(2,3-dihydroxypropyl)-acetamide]-2,4,6-trilodoisophthalamide; Omnipaque 300 contains 647 mg of iohexol equivalent to 300 mg of organic iodine per ml. The specific gravity of 1.349 of 37° C and an absolute viscosity 11.8 mPa.s at 20°C.) The particles remain in suspension for about 2-3 minutes.

Particle size uniformity and sphericity is measured using a Beckman Coulter RapidVUE Image Analyzer version 2.06 (Beckman Coulter, Miami, FL). Briefly, the RapidVUE takes an image of continuous-tone (gray-scale) form and converts it to a digital form through the process of sampling and quantization. The system software identifies and measures particles in an image in the form of a fiber, rod or sphere. Sphericity computation and other statistical definitions are in Appendix A, attached, which is a page from the RapidVUE operating manual.

Referring to FIG. 5, particle size uniformity is illustrated for particles 700 - 900 micron. The x-axis is the particle diameter. The y-axis is the volume normalized percentage of particle at each particle size. The total volume of particles detected is computed and the volume of the particles at each diameter is divided by the total volume. The embolic particles have distribution of particle sizes with variance of less than about ± 15%.

The particles can be dried by lyopholization at -20 to 20°C and a pressure of about 75 mtorr for about 30 to 70 hours. The dried particles can be rehydrated by exposure to liquid. Exposure to contrast solution indicates that rehydration achieves entry of fluid throughout the particle.

Still further enhancements are in the following claims.

## Claims

1. A drug delivery device comprising:
a spherical polymer particle having a sphericity of 90% or more, the particles having a center region from the center of the particle to a radius of r/3 including pores having a size of 20 to 35 micron and containing a therapeutic agent and a body region from r/3 to 2r/3 and a surface region from 2r/3 to r surrounding the center region, wherein the center region has a greater number of pores having a size of 20 microns to 35 microns than the body region and the surface region, and wherein the body region has a greater number of pores having a size of 5 microns to 18 microns than the surface region, and wherein the body region has a greater number of pores having a size of 5 microns to 18 microns than the surface region, wherein the particle is obtainable by a method comprising:
- generating drops comprising a base polymer and a gelling precursor;
- removing of the gelling precursor; and
- combining the particles with a therapeutic agent.

2. The device of claim 1 wherein the particle comprises PVA.

3. The device of claim 2 wherein the PVA is 1, 3 diol acetalized.

4. The device of claim 1 wherein the polymer is modified by graft polymerization.

5. The device of claim 3 wherein the particle comprises a polysaccharide.

6. The device of claim 4 wherein the polysaccharide is alginate.

7. The device of claim 1 including a coating of polymer.

8. The device of claim 7 wherein the coating is erodible.

9. The device of claim 7 including a therapeutic agent on the surface of the particle.

10. The device of claim 1 wherein the therapeutic agent is effective for the treatment of cancer.

11. The device of claim 1 wherein the particle has a diameter of 1 cm or less.

12. The device of claim 1 comprising a plurality of particles, wherein each of the plurality of particles comprises a spherical polymer particle having a sphericity of 90% or more, the particles having a center region from the center of the particle to a radius of r/3 including pores having a size of 20 microns to 35 microns and containing therapeutic agent and a body region from r/3 to 2r/3 and a surface region from 2r/3 to r. surrounding the center region, wherein the center region has a greater number of pores having a size of 20 microns to 35 microns than the body region and the surface region, and wherein the body region has a greater number of pores having a size of 5 microns to 18 microns than the surface region.

13. The device of claim 1 wherein the therapeutic agent is an anti-cancer agent.

14. A method of manufacturing a drug delivery particle according to any of claims 1 to 13, having a sphericity of 90% or more and having a porosity gradient, the particles including a center region from the center of the particle to a radius of r/3 with pores having a size of 20 to 35 micron and containing a therapeutic agent and a body region from r/3 to 2r/3 and a surface region from 2r/3 to r surrounding the center region, wherein the center region has a greater number of pores having a size of 20 microns to 35 microns than the body region and the surface region, and wherein the body region has a greater number of pores having a size of 5 microns to 18 microns than the surface region, comprising:
- generating drops comprising a base polymer and a gelling precursor;
- removing of the gelling precursor; and
- combining the particles with a therapeutic agent,

15. The method of claim 14 comprising reacting the base polymer and removing the gelling compound.

16. The method of claim 15 comprising drying the particle and exposing the dried particle to therapeutic agent.

17. The method of claim 14 comprising combining therapeutic agent prior to generating said drops.

18. The method of claim 14 wherein the gelling compound is a polysaccharide.

19. The method of claim 18 wherein the gelling compound is alginate.

20. The method of claim 14 comprising contacting the drops with a gelling agent.

21. The method of claim 20 wherein the gelling agent is a divalent cation.

22. The method of claim 14 wherein base polymer is PVA.

23. The method of claim 22 comprising reacting the PVA by acetalization.

24. The method of claim 22 wherein the PVA has a molecular weight of 75,000 g/mole or greater.

25. The method of claim 14 comprising lowering the viscosity of the base polymer and gelling compound prior to forming said drops.

26. The method of claim 25 comprising lowering the viscosity by heating.

27. The method of claim 14 comprising forming said drops by vibratory nebulization.

28. The method of claim 14 wherein the therapeutic agent is an anti-cancer agent.

29. Use of a composition, wherein said composition comprising a substantially spherical polymer particle obtainable by the method of claim 14 and having a sphericity of 90% or more, the particle comprising polyvinyl alcohol, and including a center region from the center of the particle to a radius of r/3 including pores having a size of 20 micron to 35 micron, a body region from r/3 to 2r/3 and a surface region from 2r/3 to r surrounding the center region, wherein the center region has a greater number of pores having a size of 20 microns to 35 microns than the body region and the surface region and a therapeutic agent carried by the particle, and wherein the body region has a greater number of pores having a size of 5 microns to 18 microns than the surface region for the manufacture of a medicament for a drug delivery system.

30. The use of claim 29, wherein the drug delivery system is useful for administration by percutaneous injection.

31. The use of claim 29, wherein the drug delivery system is useful for administration by a catheter.

32. The use of claim 29, wherein said system is used for treatment of uterine fibroids.

33. The use of claim 29, wherein said system is used for treatment of a cancerous lesion.

## Patentansprüche

1. Wirkstoffabgabevorrichtung umfassend:
ein sphärisches Polymerpartikel mit einer Sphärizität von 90% oder mehr, wobei die Partikel einen Zentralbereich vom Zentrum des Partikels bis zu einem Radius von r/3 umfassend Poren mit einer Größe von 20 bis 35 Mikrometer und enthaltend einen Wirkstoff und einen Körperbereich von r/3 bis 2r/3 und einen Oberflächenbereich von 2r/3 bis r aufweisen, die den Zentralbereich umgeben, wobei der Zentralbereich eine größere Anzahl von Poren mit einer Größe von 20 Mikrometer bis 35 Mikrometer aufweist als der Körperbereich und der Oberflächenbereich und wobei der Körperbereich eine größere Anzahl von Poren mit einer Größe von 5 Mikrometer bis 18 Mikrometer aufweist als der Oberflächenbereich und wobei der Körperbereich eine größere Anzahl von Poren mit einer Größe von 5 Mikrometer bis 18 Mikrometer aufweist als der Oberflächenbereich, wobei das Partikel erhältlich ist durch ein Verfahren umfassend:
- Erzeugen von Tropfen umfassend ein Basispolymer und einen Gelierungsvorläufer;
- Entfernen des Gelierungsvorläufers; und
- Kombinieren der Partikel mit einem Wirkstoff.

2. Vorrichtung gemäß Anspruch 1, wobei das Partikel PVA umfasst.

3. Vorrichtung gemäß Anspruch 2, wobei das PVA 1,3-diol acetalisiert ist.

4. Vorrichtung gemäß Anspruch 1, wobei das Polymer modifiziert ist durch Pfropfpolymerisation.

5. Vorrichtung gemäß Anspruch 3, wobei das Partikel ein Polysaccharid umfasst.

6. Vorrichtung gemäß Anspruch 4, wobei das Polysaccharid Alginat ist.

7. Vorrichtung gemäß Anspruch 1 umfassend eine Polymerbeschichtung.

8. Vorrichtung gemäß Anspruch 7, wobei die Beschichtung erodierbar ist.

9. Vorrichtung gemäß Anspruch 7 umfassend einen Wirkstoff auf der Oberfläche des Partikels.

10. Vorrichtung gemäß Anspruch 1, wobei der Wirkstoff zur Behandlung von Krebs wirksam ist.

11. Vorrichtung gemäß Anspruch 1, wobei das Partikel einen Durchmesser von 1 cm oder weniger aufweist.

12. Vorrichtung gemäß Anspruch 1 umfassend eine Vielzahl von Partikeln, wobei jedes der Vielzahl von Partikeln ein sphärisches Polymerpartikel mit einer Sphärizität von 90% oder mehr umfasst, wobei die Partikel einen Zentralbereich vom Zentrum des Partikels bis zu einem Radius von r/3 umfassend Poren mit einer Größe von 20 Mikrometer bis 35 Mikrometer und enthaltend Wirkstoff und einen Körperbereich von r/3 bis 2r/3 und einen Oberflächenbereich von 2r/3 bis r aufweisen, die den Zentralbereich umgeben, wobei der Zentralbereich eine größere Anzahl von Poren mit einer Größe von 20 Mikrometer bis 35 Mikrometer aufweist als der Körperbereich und der Oberflächenbereich und wobei der Körperbereich eine größere Anzahl von Poren mit einer Größe von 5 Mikrometer bis 18 Mikrometer als der Oberflächenbereich aufweist.

13. Vorrichtung gemäß Anspruch 1, wobei der Wirkstoff ein Antikrebsstoff ist.

14. Verfahren zum Herstellen eines Wirkstoffabgabepartikels gemäß einem der Ansprüche 1 bis 13 mit einer Sphärizität von 90% oder mehr und mit einem Porositätsgradienten, wobei die Partikel einen Zentralbereich vom Zentrum des Partikels bis zu einem Radius von r/3 mit Poren mit einer Größe von 20 bis 35 Mikrometer umfassen und enthaltend einen Wirkstoff und einen Körperbereich von r3 bis 2r/3 und einen Oberflächenbereich von 2r/3 bis r aufweisen, die den Zentralbereich umgeben, wobei der Zentralbereich eine größere Anzahl von Poren mit einer Größe von 20 Mikrometer bis 35 Mikrometer als der Körperbereich und der Oberflächenbereich aufweist und wobei der Körperbereich eine größere Anzahl von Poren mit einer Größe von 5 Mikrometer bis 18 Mikrometer als der Oberflächenbereich aufweist, umfassend:
- Erzeugen von Tropfen umfassend ein Basispolymer und einen Gelierungsvorläufer;
- Entfernen des Gelierungsvorläufers; und
- Kombinieren der Partikel mit einem Wirkstoff.

15. Verfahren gemäß Anspruch 14 umfassend das Umsetzen des Basispolymers und das Entfernen des Gelierungsmittels.

16. Verfahren gemäß Anspruch 15 umfassend das Trocknen des Partikels und das Aussetzen des getrockneten Partikels gegenüber dem Wirkstoff.

17. Verfahren gemäß Anspruch 14 umfassend das Kombinieren des Wirkstoffes vor dem Erzeugen der Tropfen.

18. Verfahren gemäß Anspruch 14, wobei das Gelierungsmittel ein Polysaccharid ist.

19. Verfahren gemäß Anspruch 18, wobei das Gelierungsmittel Alginat ist.

20. Verfahren gemäß Anspruch 14 umfassend das Inkontaktbringen der Tropfen mit einem Gelierungsmittel.

21. Verfahren gemäß Anspruch 20, wobei das Gelierungsmittel ein bivalentes Kation ist.

22. Verfahren gemäß Anspruch 14, wobei das Basispolymer PVA ist.

23. Verfahren gemäß Anspruch 22 umfassend das Umsetzen des PVA durch Acetalisierung.

24. Verfahren gemäß Anspruch 22, wobei das PVA ein Molekulargewicht von 75 000 g/Mol oder größer aufweist.

25. Verfahren gemäß Anspruch 14 umfassend das Erniedrigen der Viskosität des Basispolymers und des Gelierungsmittels vor dem Ausbilden der Tropfen.

26. Verfahren gemäß Anspruch 25 umfassend das Erniedrigen der Viskosität durch Erhitzen.

27. Verfahren gemäß Anspruch 14 umfassend das Ausbilden der Tropfen durch Vibrationsvernebelung.

28. Verfahren gemäß Anspruch 14, wobei der Wirkstoff ein Antikrebsmittel ist.

29. Verwendung einer Zusammensetzung, wobei die Zusammensetzung ein im Wesentlichen sphärisches Polymerpartikel erhältlich durch das Verfahren gemäß Anspruch 14 umfasst und mit einer Sphärizität von 90% oder mehr, wobei das Partikel Polyvinylalkohol umfasst und einen Zentralbereich vom Zentrum des Partikels bis zu einem Radius von r/3 mit Poren mit einer Größe von 20 Mikrometer bis 35 Mikrometer umfasst, einen Körperbereich von r/3 bis 2r/3 und einen Oberflächenbereich von 2r/3 bis r, die den Zentralbereich umgeben, wobei der Zentralbereich eine größere Anzahl von Poren mit einer Größe von 20 Mikrometer bis 35 Mikrometer als der Körperbereich und der Oberflächenbereich aufweist und enthaltend einen Wirkstoff, der von dem Partikel getragen wird und wobei der Körperbereich eine größere Anzahl von Poren mit einer Größe von 5 Mikrometer bis 18 Mikrometer aufweist als der Oberflächenbereich zur Herstellung eines Medikamentes für ein Wirkstoffabgabesystem.

30. Verwendung gemäß Anspruch 29, wobei das Wirkstoffabgabesystem geeignet ist zur Verabreichung durch perkutane Injektion.

31. Verwendung gemäß Anspruch 29, wobei das Wirkstoffabgabesystem geeignet ist zur Verabreichung durch einen Katheter.

32. Verwendung gemäß Anspruch 29, wobei das System verwendet wird zur Behandlung von Gebärmutterfibromen.

33. Verwendung gemäß Anspruch 29, wobei das System verwendet wird zur Behandlung einer Krebsläsion.

## Revendications

1. Un dispositif de délivrance de médicament comprenant :
une particule polymère sphérique ayant une sphéricité de 90 % ou plus, les particules ayant une région centrale à partir du centre de la particule jusqu'à un rayon de *r*/3 comprenant des pores ayant une dimension de 20 à 35 microns et contenant un agent thérapeutique et une région de corps depuis *r*/3 jusqu'à 2*r*/3 et une région de surface de 2*r*/3 à *r* entourant la région centrale, où la région centrale possède un plus grand nombre de pores ayant une dimension de 20 microns à 35 microns que la région de corps et la région de surface, et où la région de corps a un plus grand nombre de pores ayant une dimension de 5 microns à 18 microns que la région de surface, où la particule peut être obtenue par un procédé comprenant :
- la production de gouttes comprenant un polymère de base et un précurseur de gélification ;
- l'élimination du précurseur de gélification ; et
- la combinaison des particules avec un agent thérapeutique.

2. Le dispositif de la revendication 1, dans lequel la particule comprend du PVA.

3. Le dispositif de la revendication 2, dans lequel le PVA est du 1,3-diol acétalisé.

4. Le dispositif de la revendication 1, dans lequel le polymère est modifié par polymérisation-greffage.

5. Le dispositif de la revendication 3, dans lequel la particule comprend un polysaccharide.

6. Le dispositif de la revendication 4, dans lequel le polysaccharide est un alginate.

7. Le dispositif de la revendication 1, comprenant un revêtement de polymère.

8. Le dispositif de la revendication 7, dans lequel le revêtement est érodable.

9. Le dispositif de la revendication 7, comprenant un agent thérapeutique sur la surface de la particule.

10. Le dispositif de la revendication 1, dans lequel l'agent thérapeutique est efficace pour le traitement du cancer.

11. Le dispositif de la revendication 1, dans lequel la particule présente un diamètre de 1 cm ou moins.

12. Le dispositif de la revendication 1, comprenant une pluralité de particules, dans lequel chacune de la pluralité de particules comprend une particule polymère sphérique ayant une sphéricité de 90 % ou plus, les particules ayant une région centrale à partir du centre de la particule jusqu'à un rayon de *r*/3 comprenant des pores ayant une dimension de 20 à 35 microns et contenant un agent thérapeutique et une région de corps depuis *r*/3 jusqu'à 2*r*/3 et une région de surface de 2*r*/3 à *r* entourant la région centrale, où la région centrale possède un plus grand nombre de pores ayant une dimension de 20 microns à 35 microns que la région de corps et la région de surface, et où la région de corps a un plus grand nombre de pores ayant une dimension de 5 microns à 18 microns que la région de surface.

13. Le dispositif de la revendication 1, dans lequel l'agent thérapeutique est un agent anticancéreux.

14. Un procédé de fabrication d'une particule de délivrance de médicament selon l'une des revendications 1 à 13, présentant une sphéricité de 90 % ou plus et présentant un gradient de porosité, les particules ayant une région centrale à partir du centre de la particule jusqu'à un rayon de *r*/3 comprenant des pores ayant une dimension de 20 à 35 microns et contenant un agent thérapeutique et une région de corps depuis *r*/3 jusqu'à 2*r*/3 et une région de surface de 2*r*/3 à *r* entourant la région centrale, où la région centrale possède un plus grand nombre de pores ayant une dimension de 20 microns à 35 microns que la région de corps et la région de surface, et où la région de corps a un plus grand nombre de pores ayant une dimension de 5 microns à 18 microns que la région de surface, comprenant :
- la production de gouttes comprenant un polymère de base et un précurseur de gélification ;
- l'élimination du précurseur de gélification ; et
- la combinaison des particules avec un agent thérapeutique.

15. Le procédé de la revendication 14, comprenant la mise en réaction du polymère de base et l'élimination du composé gélifiant.

16. Le procédé de la revendication 15, comprenant le séchage de la particule et l'exposition de la particule séchée à l'agent thérapeutique.

17. Le procédé de la revendication 14, comprenant la combinaison de l'agent thérapeutique avant la production desdites gouttes.

18. Le procédé de la revendication 14, dans lequel le composé de gélification est un polysaccharide.

19. Le procédé de la revendication 18, dans lequel le composé de gélification est un alginate.

20. Le procédé de la revendication 14, comprenant la mise en contact des gouttes avec un agent de gélification.

21. Le procédé de la revendication 20, dans lequel l'agent de gélification est un cation bivalent.

22. Le procédé de la revendication 14, dans lequel le polymère de base est du PVA.

23. Le procédé de la revendication 22, comprenant la mise en réaction du PVA par acétalisation.

24. Le procédé de la revendication 22, dans lequel le PVA présente un poids moléculaire de 75 000 g/mole ou plus.

25. Le procédé de la revendication 14, comprenant l'abaissement de la viscosité du polymère de base et du composé de gélification avant de former lesdites gouttes.

26. Le procédé de la revendication 25, comprenant l'abaissement de la viscosité par chauffage.

27. Le procédé de la revendication 14, comprenant la formation desdites gouttes par nébulisation vibratoire.

28. Le procédé de la revendication 14, dans lequel l'agent thérapeutique est un agent anticancéreux.

29. Utilisation d'une composition, où ladite composition comprend une particule polymère substantiellement sphérique qui peut être obtenue par le procédé de la revendication 14 et qui présente une sphéricité de 90 % ou plus, la particule comprenant un alcool polyvinylique, et ayant une région centrale à partir du centre de la particule jusqu'à un rayon de *r*/3 comprenant des pores ayant une dimension de 20 à 35 microns, une région de corps depuis *r*/3 jusqu'à 2*r*/3 et une région de surface de 2*r*/3 à *r* entourant la région centrale, où la région centrale possède un plus grand nombre de pores ayant une dimension de 20 microns à 35 microns que la région de corps et la région de surface et un agent thérapeutique véhiculé par la particule, et où la région de corps a un plus grand nombre de pores ayant une dimension de 5 microns à 18 microns que la région de surface, pour la fabrication d'un médicament pour un système de délivrance de médicament.

30. L'utilisation de la revendication 29, dans laquelle le système de délivrance de médicament est utile pour une administration par injection percutanée.

31. L'utilisation de la revendication 29, dans laquelle le système de délivrance de médicament est utile pour une administration par un cathéter.

32. L'utilisation de la revendication 29, dans laquelle le système est utilisé pour le traitement des fibromes utérins.

33. L'utilisation de la revendication 29, dans laquelle le système est utilisé pour le traitement d'une lésion cancéreuse.
